# EUROPÄISCHE PATENTSCHRIFT

(11) **EP 1 642 554 B1**
(45) Veröffentlichungstag und Bekanntmachung des Hinweises auf die Patenterteilung: **21.05.2008**
(21) Anmeldenummer: 05020693.7
(22) Anmeldetag: 22.09.2005
(51) Int. Cl.: A61F 5/01

(54) **Knieorthese**
Knee brace
Orthèse du genou

(30) Priorität: 27.09.2004 DE 102004046743
(43) Veröffentlichungstag der Anmeldung: 05.04.2006
(73) Patentinhaber: Lambert, Gerhard, 5020 Salzburg (AT)
(72) Erfinder: Lambert, Gerhard, 5020 Salzburg (AT)
(74) Vertreter: Popp, Eugen

(56) Entgegenhaltungen:
- EP-A- 0 361 405
- EP-A- 0 428 497
- GB-A- 2 288 435
- US-A- 4 773 404
- US-A- 5 286 250

## Beschreibung

Die Erfindung betrifft eine Knieorthese mit einem femuralen und einem tibialen Orthesenteil gemäß dem Oberbegriff des Patentanspruchs 1.

Knieorthesen der genannten Art werden therapeutisch sowie postoperativ zur Unterstützung und Sicherung des Gelenksystems am Knie verwendet. Die Orthese, die hierzu mittels wenigstens einer Manschette sowohl am Oberschenkel als auch am Unterschenkel befestigt wird, weist ein Gelenksystem auf, das einen anatomisch korrekten Bewegungsablauf des Kniegelenks nachahmen soll. Wesentlich ist hierbei, daß das Kniegelenk, das üblicherweise nach einer Verletzung oder einer Operation geschwächt oder geschädigt ist, nicht durch Inkongruenzen zwischen der funktionellen Anatomie des menschlichen Kniegelenks und der technischen Gelenkführung durch die Knieorthese mechanisch gestreßt wird. Solche Inkongruenzen können, sofern eine anatomisch korrekte Führung des Orthesengelenks nicht gewährleistet ist, zu Scher-, Stauch- und/oder Zugbeanspruchungen des menschlichen Kniegelenks führen, was, besonders wenn die Orthese fest am Bein verankert ist, im schlimmsten Fall zu einer weiteren Schädigung des Kniegelenks führt, was es unbedingt zu vermeiden gilt.

Eine anatomisch und funktionell korrekte Nachahmung des menschlichen Beugebewegungsablaufs am Knie ist deshalb äußerst schwierig, weil die Circumferenz des femuralen Kondylus doppelt so lang ist wie die tibiale Gelenksfläche, so daß eine reine Abrollbewegung des Gelenks unmöglich ist. So zeigen Untersuchungen der realen Bewegungsabläufe zwischen dem femuralen und dem tibialen Kondylus, daß der femurale Kondylus auf der Tibia rollt und gleitet, so daß eine posteriore Luxation verhindert wird. Bei der Beugung wird hierbei gezielt das vordere Kreuzband beansprucht, das den femuralen Kondylus nach anterior zieht bzw. anterior hält. Dies bedeutet mit anderen Worten, daß bei der Beugung das vordere Kreuzband für das nach vorne Gleiten des femuralten Kondylus verantwortlich ist, während das hintere Kreuzband bei einer Streckung posterior in analoger Weise wirkt.

Um diesen äußerst komplexen Bewegungsablauf nachzuahmen, werden für Kniegelenke polyzentrische Gelenke verwendet, mittels derer es möglich ist, die vorgenannten Inkongruenzen zwischen der funktionellen Anatomie des menschlichen Kniegelenks und der technischen Gelenkführung der Knieorthese zu vermeiden. Diese polyzentrischen Gelenke weisen keinen fest definierten Drehpunkt, sondern einen sich im Verlauf einer Beuge- bzw. Streckbewegung des Beins ständig in seiner Lage verändernden Drehpunkt auf. Somit ist der jeweilige Momentan-Drehpunkt des Kniegelenks, respektive des die Beugebewegung des Knies nachahmenden Orthesengelenks, in seiner Lage im zwei- bzw. dreidimensionalen Raum vom Beugewinkel des Gelenks abhängig, wobei die Vielzahl der jeweiligen Drehpunkte eine Kurve, die auch als Zentrode bezeichnet wird, beschreibt.

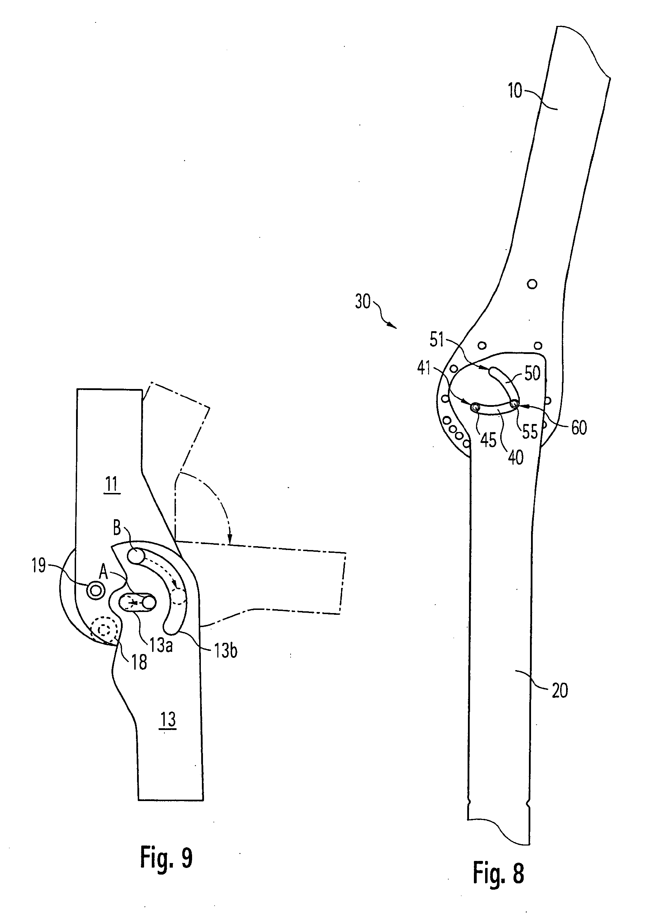

Eine Realisierung eines solchen polyzentrischen Gelenksystems ist beispielsweise in der EP 0 361 405 B1 und der damit korrespondierenden DE 689 28 511 T2 beschrieben, die sich mit einer Knieorthese mit einer gesteuerten Mehrachsenbewegung befaßt. Der dortige Gelenkmechanismus, der in Fig. 9 dargestellt ist, umfaßt zwei Nockenschlitze 13a, 13b und zwei Nockenstiftfolger A, B, die an einander zugeordneten Gelenkteilen 13, 11 befestigt sind, wobei der eine Nockenschlitz 13a in einer Querebene angeordnet ist und dazu dient, die Vorwärtsbewegung des oberen Gelenkteils 11 zu ermöglichen, während der zweite Nockenschlitz 13b in einer Längsorientierung angeordnet ist und ein langes Bogensegment für eine unizentrische Phase der Gelenkarthrokinematik zur Verfügung stellt. Zu Beginn einer Beugebewegung bewegt sich bei dortigem Gelenkmechanismus der obere Nockenstiftfolger B in dem sich in Längsrichtung erstreckenden gebogenen Schlitz 13b nach unten vorne. Im Verlauf dieser Bewegung nähert sich der obere Nockenstiftfolger B dem unteren in der horizontalen Querebene angeordneten Nockenschlitz 13a, wobei sich der in diesem unteren Nockenschlitz 13a geführte Nockenstiftfolger A nach hinten verlagert.

Nachteilig bei der dortigen Konstruktion ist die Tatsache, daß sich bei einer Beugung der femur- und tibiaseitigen Orthesenteile 11, 13 gleichzeitig beide Nockenstiftfolger A, B in den zugehörigen Nockenschlitzen 13a, 13b bewegen, wobei die Bewegungen der Nockenstiftfolger A, B in unterschiedliche Richtungen erfolgen, die durch die Nockenschlitze 13a, 13b vorgegeben sind. Die exakte Einhaltung der Bahnen der Nockenstiftfolger A, B in den Nockenschlitzen 13a, 13b ist nur möglich, indem die Nockenstiftfolger A, B seitlich an den Begrenzungen der Nockenschlitze 13a, 13b entlang gleiten, wobei eine bewegungshemmende Reibung der Nockenstiftfolger A, B an den Rändern der Nockenschlitze 13a, 13b mit einer damit einhergehenden Abnutzung der Nockenstiftfolger A, B und der Ränder auftritt. Des weiteren ist aus Fig. 9 deutlich zu erkennen, daß der mit A bezeichnete untere, in der horizontalen Querebene verlaufende Nockenstiftfolger bei einer aus der gestreckten Lage des Beins beginnenden Beugung zunächst in die in der Zeichnung dargestellte rechte untere Ecke des horizontalen Nockenschlitzes 13a gedrängt wird, wobei ein hoher Druck auf die untere Kante des Nockenschlitzes 13a ausgeübt wird, so daß eine Sperrung der Beugung zu befürchten ist. Diese Problematik verstärkt sich mit zunehmender Verwendung der dort beschriebenen Knieorthese, da der Verschleiß aufgrund der fortgesetzten Reibung in sich verstärkender Weise zunimmt. Somit ist die Funktionsfähigkeit der in der EP 0 361 405 B1 beschriebenen Knieorthese nicht dauerhaft gewährleistet, wobei ein relativ häufiger Austausch des dortigen Gelenkmechanismus notwendig ist.

Eine Knieorthese der genannten Art ist in EP-A-0 428 497 beschrieben. Der Oberbegriff des Anspruchs 1 stützt sich auf dieses Dokument.

Der Erfindung liegt die Aufgabe zugrunde, eine Knieorthese mit einer polyzentrischen Gelenkführung zur Verfügung zu stellen, mittels der ein anatomisch korrekter Beugeverlauf sichergestellt und die vorgenannten Probleme bei einer gleichzeitigen kostengünstigen Herstellbarkeit vermieden werden.

Diese Aufgabe wird durch eine Knieorthese gemäß dem Patentanspruch 1 gelöst.

Insbesondere wird die Aufgabe durch eine Knieorthese mit einem femuralen und einem tibialen Orthesenteil gelöst, die über seitliche Gelenke miteinander verbunden sind, wobei die Abwinklung der Gelenke jeweils durch zwei Führungsbahnen, insbesondere Führungsschlitze, einerseits und diesen jeweils zugeordnete Nocken, insbesondere Stiftfolger, andererseits definiert ist, wobei die Führungsbahnen, insbesondere Führungsschlitze, sich längs zweier sich in einem Punkt schneidender Rastpolkurven, insbesondere Rastpolkreise, erstrecken.

Ein wesentlicher Punkt der Erfindung liegt darin, daß die Führungsschlitze als Rastpolkreise mit einem einheitlichen Radius ausgebildet sind, entlang derer sich jeweils der dem Rastpolkreis zugeordnete Stiftfolger bewegt.

Gemäß einer Ausführungsform erstrecken sich die beiden Führungsbahnen, insbesondere Führungsschlitze, unter Ausbildung einer durchgehenden triangelförmigen Nockenführung jeweils bis zum Schnittpunkt der zugeordneten Polkurven. Des weiteren liegen dem Schnittpunkt der Polkurven abgewandte Enden der beiden Führungsbahnen, insbesondere Führungsschlitze, und der Schnittpunkt der Polkurven an den Ecken eines gedachten gleichseitigen Dreiecks, wobei der Abstand der den jeweiligen Führungsbahnen zugeordneten Nocken, insbesondere Stiftfolgern, der Schenkellänge des gedachten gleichseitigen Dreiecks entspricht.

Auf diese äußerst einfache Weise ist gewährleistet, daß bei einer Beugung des Orthesengelenks jeweils nur ein Stiftfolger auf einer konzentrischen Kreisbahn, nämlich dem der Führungsbahn zugeordneten Abschnitt eines Rastpolkreises, bewegt wird, wobei der jeweils andere Stiftfolger den jeweiligen Drehpunkt bildet. Ein gegenseitiges Sperren der Stiftfolger ist somit sicher ausgeschlossen.

Da der Bewegungsablauf des in dem Führungsschlitz bewegten Stiftfolgers durch seine Verbindung mit dem Orthosenteil einerseits und dem als Drehpunkt dienenden anderen Stiftfolger andererseits genau definiert ist, ist eine Anlage des bewegten Stiftfolgers an den Rändern des Führungsschlitzes nicht zwingend notwendig, so daß ein Verschleiß des Stiftfolgers sowie der Ränder vermieden wird.

Im Verlauf der Beugung des Orthesengelenks dient der eine Stiftfolger solange als Drehpunkt für die Bewegung des anderen Stiftfolgers entlang des Führungsschlitzes, bis der bewegte Stiftfolger ausgehend vom Schnittpunkt der beiden Führungsschlitze das jeweilige Ende des dem Stiftfolger zugeordneten Führungsschlitzes erreicht, woraufhin der andere, bisher als Drehpunkt dienende Stiftfolger sich nunmehr vom Ende des diesem zugeordneten Führungsschlitzes bis zum Schnittpunkt der Polkurven bewegt.

Da die beiden Führungsschlitze durch Segmente von Rastpolkreisen mit jeweils identischem Radius gebildet werden, deren jeweiliger Mittelpunkt durch einen Endbereich des jeweils gegenüberliegenden Führungsschlitzes definiert wird, ist ein hinsichtlich der Stiftfolger und der Führungsschlitze nahezu reibungsfreier harmonischer Bewegungsverlauf möglich und vorgegeben, der einem anatomisch korrekten Beugebewegungsablauf des menschlichen Knies entspricht.

Da in jeder Phase der Beugebewegung jeweils nur ein Stiftfolger in dem ihm zugeordneten Führungsschlitz bewegt wird, ist ein Versperren, Verhaken oder Verkanten wie gemäß dem in Fig. 9 dargestellten Stand der Technik erfindungsgemäß nicht möglich.

Gemäß einer Ausführungsform der Erfindung liegt der Schnittpunkt der den beiden Führungsbahnen zugeordneten Polkurven anterior, wobei die sich durch den Schnittpunkt hindurch erstreckende Winkelhalbierende der entlang des gedachten gleichseitigen Dreiecks liegenden Polkurven zwischen den beiden Führungsbahnen in Richtung von posterior nach anterior nach unten geneigt ist. Auf diese Weise wird ein aufrechtes Stehen sowie ein Überstrecken des Beins bis zu einer Hyperextension von bis zu -10° ermöglicht.

Des weiteren schließt die Winkelhalbierende mit der Längsachse der gestreckten Knieorthese an der anterioren femuralen Seite einen Winkel (α) von 100° bis 120°, insbesondere einen Winkel von etwa 110°, ein.

Die beiden Führungsbahnen der Stiftfolger erstrecken sich erfindungsgemäß über einen Bogenwinkel β von bis zu 60°, so daß eine übergangsloser Drehpunktverlagerung möglich ist. Diese Drehpunktverlagerung findet in vorteilhafter Weise dann statt, wenn das Kniegelenk bezüglich maximaler Streckung und maximaler Beugung zur Hälfte gebeugt ist. Somit findet die Verlagerung des Drehpunkts in einer Stellung minimaler Belastung des Kniegelenks statt, was zu einer besonderen Leichtgängigkeit und Verschleißfreiheit des erfindungsgemäßen Orthesengelenks maßgeblich beiträgt.

Wie bereits vorerwähnt, ist mit der erfindungsgemäßen Knieorthese eine Hyperextension von bis zu -10° möglich. Dies wird dadurch erreicht, daß die Lage der beiden Führungsbahnen bzw. der diesen zugeordneten Nocken so aufeinander abgestimmt ist, daß die Verbindungslinie zwischen den beiden Nocken, insbesondere Stiftfolgern, in gestrecktem Zustand in Richtung von posterior nach anterior nach oben geneigt ist. Die Neigung, die mit einem Winkel γ ausgedrückt ist, beträgt vorzugsweise 5° ≤ γ ≤ 20°. Gemäß einer bevorzugten Ausführungsform beträgt der Winkel γ etwa 8° bis 12°, insbesondere 10°.

Des weiteren sei erwähnt, daß die Führungsbahnen bzw. die durchgehende Nockenführung Teil einer dem femuralen oder tibialen Orthesenteil zugeordneten ersten Schiene und die Nocken Teil einer dem jeweils anderen Orthesenteil zugeordneten zweiten Schiene ist. Dies bedeutet mit anderen Worten, daß die Nockenführung entweder, wie erfindungsgemäß in den Zeichnungen dargestellt, am tibialen Orthesenteil vorgesehen sein kann, während die Nocken am femuralen Orthesenteil angebracht sind. Die erfindungsgemäße Funktion der Knieorthese ist jedoch auch gegeben, wenn die Nockenführung am femuralen Orthesenteil und die jeweils zugeordneten Nocken am tibialen Orthesenteil angebracht sind. Somit ist die Funktionalität der erfindungsgemäßen Knieorthese schwerkraftunabhängig und insofern auch bei liegenden Patienten oder Patienten mit hochgelagertem Bein anwendbar, ohne daß ein Verhaken oder ungewolltes Einrasten zu befürchten ist.

Des weiteren sind dem erfindungsgemäßen Orthesengelenk posteriore und/oder anteriore Anschlagmittel zur Begrenzung der Abwinklung und/oder der Streckung zugeordnet. Diese bestehen gemäß einer Ausführungsform der Erfindung aus einfachen Bohrungen, die in den jeweiligen Orthesenteilen vorgesehen sind und die Durchführung eines Begrenzungsstiftes ermöglichen, an dem die jeweiligen Außenkanten der femuralen bzw. tibialen Orthesenteile anliegen. Die Bohrungen sind so angebracht, daß praktisch alle von einem Orthopäden als sinnvoll erachtete Begrenzungswinkel für die Abwinklung und/oder die Streckung fixiert werden können bzw. eine Beugung oder Streckung des Kniegelenks nur innerhalb vorgegebener Grenzen möglich ist, so daß die Knieorthese individuell auf unterschiedlichste Stützfunktionen eingestellt werden kann.

Im übrigen ist an den Gelenken zumindest eine seitliche äußere Abdeckung vorgesehen, die insbesondere mittels einer Schnapp-, Hak- oder Rasthalterung ausgestattet ist. Hierfür kommen beispielsweise Druckknöpfe oder auch eine einfache Klettverbindung in Frage.

Weitere Ausführungsformen der Erfindung ergeben sich aus den Unteransprüchen.

Nachfolgend wird die Erfindung anhand eines Ausführungsbeispiels beschrieben, das anhand der Abbildungen näher erläutert wird. Hierbei zeigen:
- Fig. 1: eine schematische Darstellung eines tibialen Orthesenteils;
- Fig. 2: eine schematische Darstellung eines femuralen Orthesenteils;
- Fig. 3-8: eine schematische Darstellung einer erfindungsgemäßen Knieorthese mit unterschiedlichen Beugungswinkeln; und
- Fig. 9: ein Knieorthesengelenk gemäß dem Stand der Technik.

In der nachfolgenden Beschreibung werden für gleiche und gleich wirkende Teile dieselben Bezugsziffern verwendet.

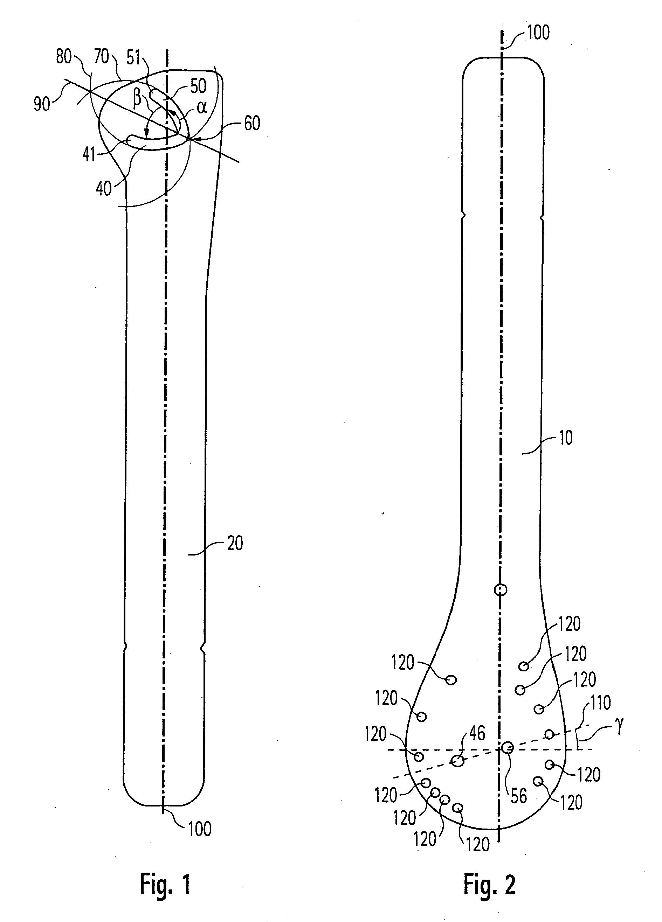

Fig. 1 zeigt ein tibiales Orthesenteil 20 mit einer Längsachse 100 sowie zwei Führungsschlitzen 40 und 50. Die Führungsschlitze 40 und 50 erstrecken sich über einen Abschnitt eines jeweils zugeordneten Rastpolkreises 70, 80 und schneiden sich in einem Schnittpunkt 60. Auf der dem Schnittpunkt 60 abgewandten Seite der Führungsschlitze 40, 50 befinden sich deren jeweilige Enden 41, 51, die als Anschlag und Drehpunkt für die in den Führungsschlitzen 40, 50 laufenden Nocken, insbesondere Stiftfolger 45, 55 dienen. Durch die Schnittpunkte der Rastpolkreise 70, 80 sowie durch den Schnittpunkt 60 der Führungsschlitze 40, 50 erstreckt sich eine Winkelhalbierende 90. Der Winkel α der Winkelhalbierenden 90 bezüglich der Längsachse der Knieorthese 100 beträgt 110°. Zwischen den beiden Führungsbahnen, bzw. Führungsschlitzen 40, 50 ist in Fig. 1 ein Bogenwinkel β eingezeichnet. Dieser beträgt 60° und definiert jeweils eine Seite eines gleichseitigen Dreiecks.

In Fig. 2 ist das dem tibialen Orthesenteil aus Fig. 1 zugeordnete femurale Orthesenteil 10 mit eingezeichneter Längsachse 100 der Knieorthese dargestellt. Das femurale Orthesenteil weist zwei Bohrungen 46, 56 für die Anbringung von Nocken bzw. Stiftfolgern 45, 55 auf, die entlang einer Nockenverbindungslinie 110 angeordnet sind. Die Nockenverbindungslinie 110 schließt mit einer senkrecht zur Längsachse 100 der Knieorthese liegenden horizontalen Linie einen Winkel γ von ca. 15° ein. Ferner weist das femurale Orthesenteil 10 Anschlagmittel 120 in Form von Bohrungen auf, durch die ein Begrenzungsstift (nicht gezeigt) geführt und in diesen arretiert werden kann. In diesem Zusammenhang sei erwähnt, daß zur Bildung der Knieorthese in vorteilhafter Weise auf jeder Seite der Orthese zwei femurale Orthesenteile 10 vorgesehen sind, zwischen denen das tibiale Orthesenteil 20 mit Hilfe der Führungsschlitze 40, 50 und der Stiftfolger 45, 55 geführt wird, so daß ein Begrenzungsstift, der durch eines der Anschlaglöcher 120 gesteckt wird, nach einer Vorbeiführung an dem tibialen Orthesenteil in einem weiteren Anschlagloch 120 eines zweiten zugeordneten femuralen Orthesenteils 10 gehaltert wird und somit mit seinen beiden Enden in jeweils einem Anschlagloch 120 verankert ist.

In vorteilhafter Weise weist die Knieorthese beidseitig eine Gelenkkonstruktion bestehend aus zwei femuralen 10 und einem tibialen Orthesenteil 20 auf, wobei bei entsprechend steifer Ausführung der Knieorthese auch eine einseitige Gelenkführung gegebenenfalls zur Stabilisierung des Kniegelenks und zur Führung desselben ausreicht.

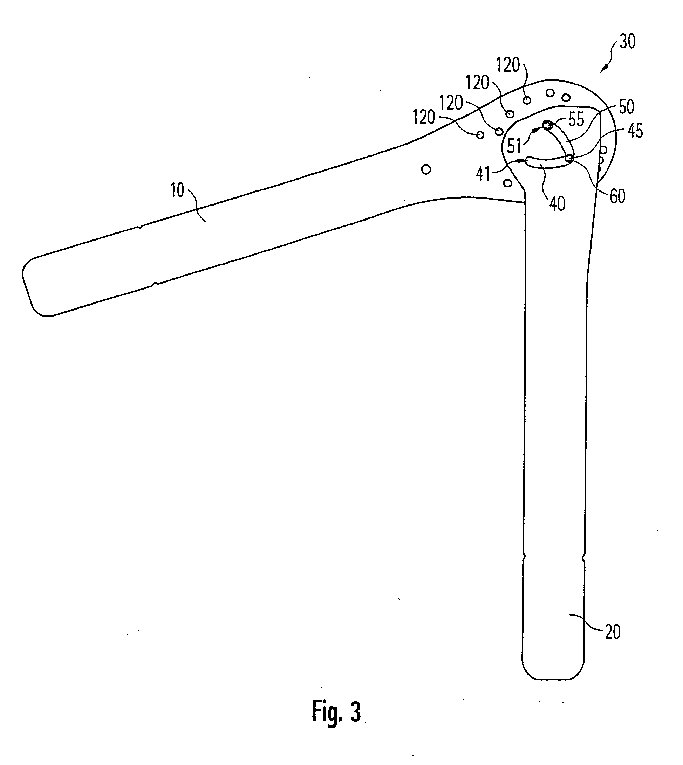

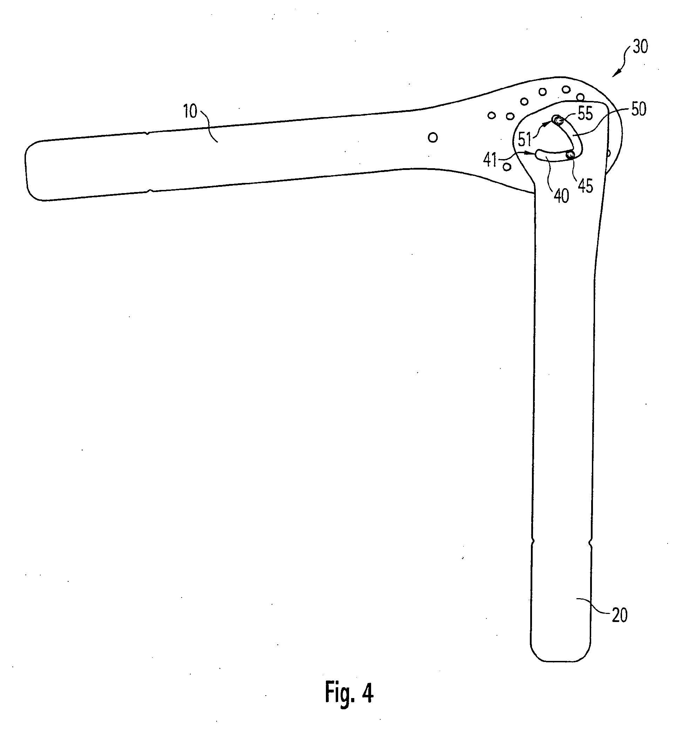

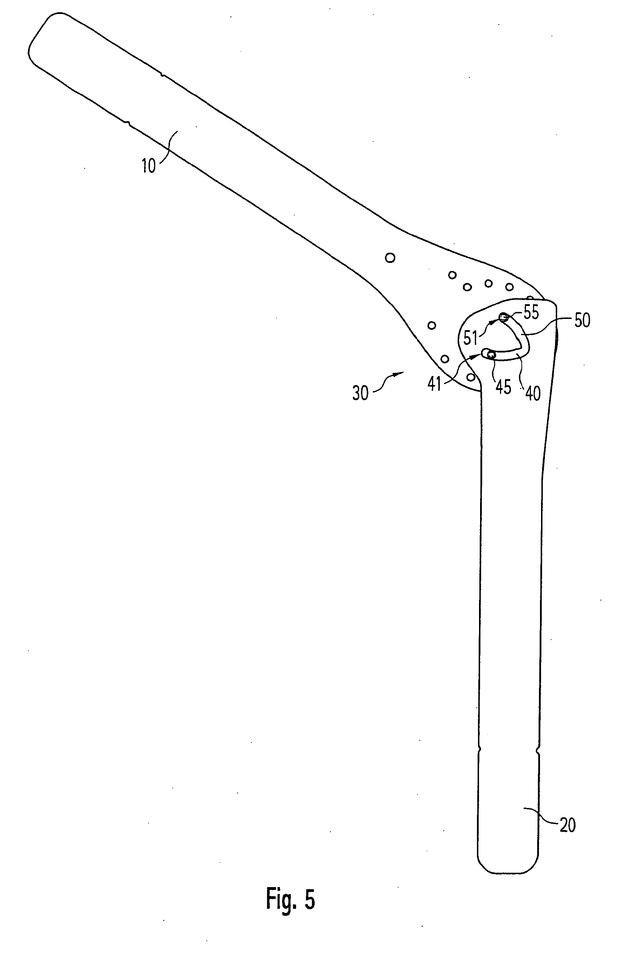

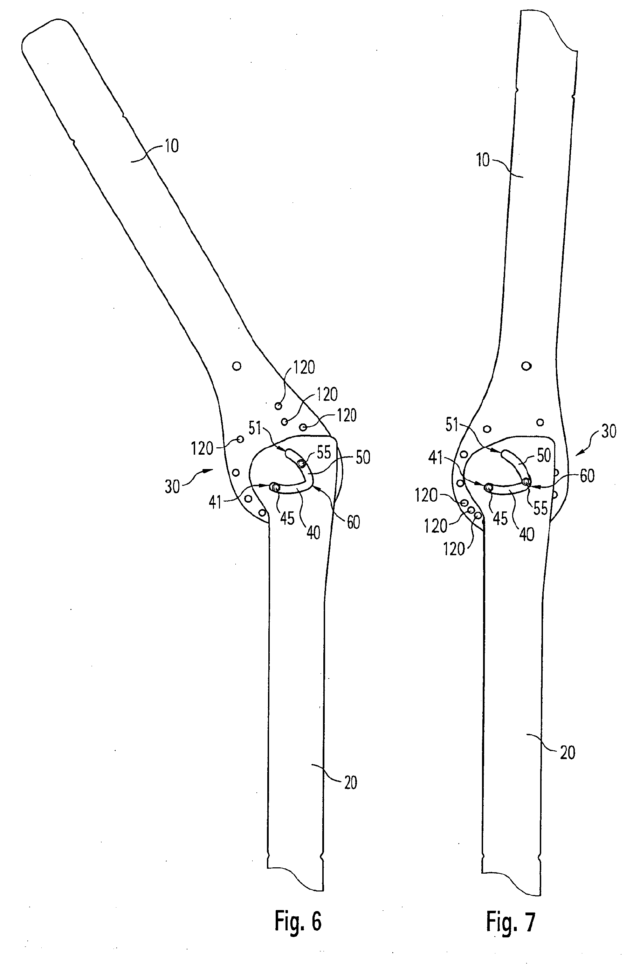

Die Figuren 3 bis 8 zeigen eine schematische Darstellung eines erfindungsgemäßen Knieorthesengelenks 30 in Teilschnittansicht, wobei an einem femuralen Orthesenteil 10 zwei Stiftfolger 45, 55 angebracht sind, die in, an einem tibialen Orthesenteil vorgesehenen, Führungsschlitzen 40, 50 verlaufen. Gemäß Fig. 3 ist das Orthesengelenk 30 mit seinem maximalen Beugewinkel dargestellt, bei dem der Stiftfolger 55 im Ende 51 des Führungsschlitzes 50 zu liegen kommt, während der Stiftfolger 45 im Schnittpunkt 60 der beiden Führungsschlitze 40, 50 angeordnet ist. Wie in den Figuren 4, 5 und 6 zu erkennen ist, verlagert sich der Stiftfolger 45 bei einer Streckung des Orthesengelenks 30 entlang des Führungsschlitzes 40 nach hinten in Richtung Ende 41 des Führungsschlitzes 40, während der Stiftfolger 55, der im Ende 51 des Führungsschlitzes 50 angeordnet ist, als Drehpunkt für die Bewegung des Stiftfolgers 45 entlang des Abschnitts des Rastpolkreises 80 dient, entlang dessen der Führungsschlitz 40 ausgebildet ist.

Im Zustand des halben maximalen Beugewinkels der Knieorthese sind beide Stiftfolger 45 und 50 in den jeweils zugeordneten Enden 41, 51 der jeweiligen Führungsschlitze 40, 50 angeordnet. Bei einer weiteren Streckung des Orthesengelenks 30 fungiert nun der im Ende 41 des Führungsschlitzes 40 angeordnete Stiftfolger 45 als Drehpunkt, wobei sich im Verlauf der weiteren Streckung der Stiftfolger 55 entlang des rastpolkreisförmigen Führungsschlitzes 50 bis zum Schnittpunkt 60 der Führungsschlitze 40, 50 bewegt. Da die Winkelhalbierende 90 zwischen den Führungsschlitzen 40, 50 einerseits nach unten geneigt ist und sich die Verbindungslinie 110 zwischen den Stiftfolgern 45, 55 in gestrecktem Zustand in Richtung von posterior nach anterior nach oben erstreckt, ist eine Hyperextension von bis zu -10° möglich. Eine solche Hyperextension ist in Fig. 8 dargestellt.

### Bezugszeichen

- 10: femurales Orthesenteil
- 20: tibiales Orthesenteil
- 30: Gelenk
- 40: Führungsschlitz
- 41: Ende des Führungsschlitzes
- 45: Stiftfolger
- 46: Bohrung für Stiftfolger
- 50: Führungsschlitz
- 55: Stiftfolger
- 56: Bohrung für Stiftfolger
- 60: Schnittpunkt
- 70: Rastpolkreis
- 80: Rastpolkreis
- 90: Winkelhalbierende
- 100: Längsachse der Knieorthese
- 110: Nockenverbindungslinie
- 120: Anschlagmittel

## Patentansprüche

1. Knieorthese mit einem femuralen (10) und einem tibialen (20) Orthesenteil, die über seitliche Gelenke (30) miteinander verbunden sind, wobei die Abwinklung der Gelenke (30) jeweils durch zwei Führungsschlitze (40, 50) einerseits und diesen jeweils zugeordnete Nocken (45, 55), andererseits definiert ist,
**dadurch gekennzeichnet, daß**
die Führungsschlitze (40, 50) sich längs zweier sich in einem Punkt (60) schneidender Rastpolkreise (70, 80) erstrecken und eine durchgehende triangelförmige Nockenführung bilden, die sich jeweils bis zum Schnittpunkt (60) der zugeordneten Polkurven erstreckt.

2. Knieorthese nach Anspruch 1,
**dadurch gekennzeichnet, daß**
dem Schnittpunkt (60) der Polkurven abgewandte Enden (41, 51) der beiden Führungsschlitze (40, 50), und der Schnittpunkt (60) an den Ecken eines gedachten gleichseitigen Dreiecks liegen.

3. Knieorthese nach Anspruch 3,
**dadurch gekennzeichnet, daß**
der Abstand zwischen den beiden Nocken (45, 55), der Schenkellänge des gedachten gleichseitigen Dreiecks entspricht.

4. Knieorthese nach einem der vorhergehenden Ansprüche,
**dadurch gekennzeichnet, daß**
der Schnittpunkt (60) der den beiden Führungsschlitze zugeordneten Polkurven anterior liegt, und daß die sich durch den Schnittpunkt (60) hindurch erstreckende Winkelhalbierende (90) zwischen den beiden Führungsschlitze in Richtung von posterior nach anterior nach unten geneigt ist.

5. Knieorthese nach Anspruch 4,
**dadurch gekennzeichnet, daß**
die Winkelhalbierende (90) mit der Längsachse (100) der gestreckten Knieorthese (15) an der anterioren Seite einen Winkel (α) von 100° bis 120°, insbesondere etwa 110°, einschließt.

6. Knieorthese nach einem der vorhergehenden Ansprüche,
**dadurch gekennzeichnet, daß**
die beiden Führungsschlitze (40, 50) sich über einen Bogenwinkel (β) von bis zu 60° erstrecken.

7. Knieorthese nach einem der vorhergehenden Ansprüche,
**dadurch gekennzeichnet, daß**
die Lage der beiden Führungsschlitze (40, 50) bzw. der diesen zugeordneten Nocken (45, 55) derart ist, daß eine Hyperextension von bis zu -10° möglich ist.

8. Knieorthese nach einem der vorhergehenden Ansprüche,
**dadurch gekennzeichnet, daß**
die Verbindungslinie (110) zwischen den beiden Nocken (45, 55) in gestrecktem Zustand in Richtung von posterior nach anterior nach oben geneigt ist, insbesondere in einem Winkel (γ) von etwa 5° bis 20°, insbesondere etwa 10°.

9. Knieorthese nach einem der vorhergehenden Ansprüche,
**dadurch gekennzeichnet, daß**
die Führungsschlitze (40, 50) bzw. durchgehende Nockenführung Teil einer dem femuralen (10) oder tibialen (20) Orthesenteil zugeordneten ersten Schiene und die Nocken (45, 55) Teil einer dem jeweils anderen Orthesenteil zugeordneten zweiten Schiene ist.

10. Knieorthese nach einem der vorhergehenden Ansprüche,
**dadurch gekennzeichnet, daß**
wenigstens einem Gelenk posteriore und/oder anteriore Anschlagmittel (120) zur Begrenzung der Abwinklung und/oder Streckung zugeordnet sind.

11. Knieorthese nach einem der vorhergehenden Ansprüche,
**dadurch gekennzeichnet, daß**
den Gelenken zumindest eine seitliche äußere Abdeckung, insbesondere mittels Schnapp-, Hak- oder Rasthalterung, zugeordnet sind.

## Claims

1. Knee orthosis having a femoral orthosis member (10) and a tibial orthosis member (20), which are joined to one another by way of lateral joints (30), the bending of the joints (30) being defined in each case by two guide slots (40, 50) on the one hand and by associated cams (45, 55) on the other hand,
**characterized in that**
the guide slots (40, 50) extend along two fixed centrode circles (70, 80) that intersect at a point (60) and form a continuous triangular cam guide means which in each case extends as far as the intersection point (60) of the associated centrodes.

2. Knee orthosis according to claim 1,
**characterized in that**
the ends (41, 51) of the two guide slots (40, 50) remote from the intersection point (60) of the centrodes, and the intersection point (60) are located at the corners of a notional equilateral triangle.

3. Knee orthosis according to claim 2,
**characterized in that**
the distance between the two cams (45, 55) corresponds to the side length of the notional equilateral triangle.

4. Knee orthosis according to any one of the preceding claims,
**characterized in that**
the intersection point (60) of the centrodes associated with the two guide slots is located anteriorly, and the angle-bisecting line (90) passing through the intersection point (60) is inclined downwards in the direction from posterior to anterior between the two guide slots.

5. Knee orthosis according to claim 4,
**characterized in that**
the angle-bisecting line (90) encloses with the longitudinal axis (100) of the extended knee orthosis (15) on the anterior side an angle (α) of from 100° to 120°, especially approximately 110°.

6. Knee orthosis according to any one of the preceding claims,
**characterized in that**
the two guide slots (40, 50) extend over an arc angle (β) of up to 60°.

7. Knee orthosis according to any one of the preceding claims,
**characterized in that**
the position of the two guide slots (40, 50), and of the cams (45, 55) associated therewith, is such that hyperextension of up to -10° is possible.

8. Knee orthosis according to any one of the preceding claims,
**characterized in that**
the connecting line (110) between the two cams (45, 55) in the extended state is inclined upwards in the direction from posterior to anterior, especially at an angle (γ) of approximately from 5° to 20°, especially approximately 10°.

9. Knee orthosis according to any one of the preceding claims,
**characterized in that**
the guide slots (40, 50), or the continuous cam guide means, form part of a first splint associated with the femoral (10) or tibial (20) orthosis member and the cams (45, 55) form part of a second splint associated with the respective other orthosis member.

10. Knee orthosis according to any one of the preceding claims,
**characterized in that**
at least one joint is associated with posterior and/or anterior stop means (120) for limiting bending and/or extension.

11. Knee orthosis according to any one of the preceding claims,
**characterized in that**
the joints are associated with at least one lateral outer covering, especially by means of a snap fastening, hook fastening or interlocking fastening.

## Revendications

1. Orthèse du genou comportant une partie fémorale (10) et une partie tibiale (20) reliées entre elles par des articulations latérales (30), le pliage de chaque articulation (30) étant défini par deux fentes de guidage (40, 50), d'une part, et des cames (45, 55) associées auxdites fentes, d'autre part,
**caractérisée en ce que**
les fentes de guidage (40, 50) s'étendent le long de deux bases centroïdes fixes (70, 80) se coupant en un point (60) et forment un guidage de came continu de forme triangulaire, qui s'étend chaque fois jusqu'au point d'intersection (60) des centroïdes associés.

2. Orthèse du genou selon la revendication 1,
**caractérisée en ce que**
les extrémités (41, 51) des deux fentes de guidage (40, 50) qui sont situées à l'opposé du point d'intersection (60) des centroïdes, et le point d'intersection (60) se trouvent aux sommets d'un triangle équilatéral imaginaire.

3. Orthèse du genou selon la revendication 2,
**caractérisée en ce que**
la distance entre les deux cames (45, 55) équivaut à la longueur du côté du triangle équilatéral imaginaire.

4. Orthèse du genou selon l'une quelconque des revendications précédentes,
**caractérisée en ce que**
le point d'intersection (60) des centroïdes associés aux deux fentes de guidage est orienté vers l'avant, et **en ce que** la bissectrice (90) de l'angle formé par les deux fentes de guidage passant par le point d'intersection (60) est inclinée vers le bas dans le sens postéro-antérieur.

5. Orthèse du genou selon la revendication 4,
**caractérisée en ce que**
en position d'extension de l'orthèse du genou (15), la bissectrice (90) forme avec l'axe longitudinal (100), sur le côté antérieur, un angle (α) compris entre 100° et 120° et notamment sensiblement égal à 110°.

6. Orthèse du genou selon l'une quelconque des revendications précédentes,
**caractérisée en ce que**
les deux fentes de guidage (40, 50) forment un angle (β) inférieur ou égal à 60°.

7. Orthèse du genou selon l'une quelconque des revendications précédentes,
**caractérisée en ce que**
la position des deux fentes de guidage (40, 50) et/ou des cames (45, 55) qui leur sont associées est propre à permettre une hyperextension pouvant aller jusqu'à - 10°.

8. Orthèse du genou selon l'une quelconque des revendications précédentes,
**caractérisée en ce que**
à l'état d'extension, la ligne de jonction (110) entre les deux cames (45, 55) est inclinée vers le haut dans le sens postéro-antérieur, notamment selon un angle (γ) compris entre environ 5° et 20°, et notamment égal à environ 10°.

9. Orthèse du genou selon l'une quelconque des revendications précédentes,
**caractérisée en ce que**
les fentes de guidage (40, 50) ou le guidage continu des cames font partie d'une première attelle associée à la partie fémorale (10) ou tibiale (20) de l'orthèse, les cames (45, 55) faisant partie d'une seconde attelle associée à l'autre partie de l'orthèse.

10. Orthèse du genou selon l'une quelconque des revendications précédentes,
**caractérisée en ce que**
des moyens de butée postérieurs et/ou antérieurs (120) sont associés à au moins une articulation pour limiter le pliage et/ou l'extension.

11. Orthèse du genou selon l'une quelconque des revendications précédentes,
**caractérisée en ce que**
au moins un cache latéral externe est associé aux articulations, le ou lesdits caches étant maintenus notamment au moyen de crochets ou par enclipsage ou par encliquetage.
